(19) Europäisches Patentamt
European Patent Office
Office européen des brevets

(11) Numéro de publication: 0 053 978
B1

(12) # FASCICULE DE BREVET EUROPEEN

(45) Date de publication du fascicule du brevet: **02.11.83**

(51) Int. Cl.³: **C 07 J 1/00**, A 61 K 31/565, A 23 K 1/165

(21) Numéro de dépôt: **81401919.6**

(22) Date de dépôt: **03.12.81**

(54) **Nouveaux dérivés de l'estra 4,9-diène, leur procédé de préparation et leur application comme médicaments.**

(30) Priorité: **05.12.80 FR 8025868**

(43) Date de publication de la demande: **16.06.82 Bulletin 82/24**

(45) Mention de la délivrance du brevet: **02.11.83 Bulletin 83/44**

(84) Etats contractants désignés: **BE DE GB IT LU NL**

(56) Documents cités:
**FR - A - 2 377 418**
**US - A - 3 086 027**
**US - A - 3 325 520**

(73) Titulaire: **ROUSSEL-UCLAF, 35, boulevard des Invalides, F-75007 Paris (FR)**

(72) Inventeur: **Teutsch, Jean, Georges, Résidence Lavoisier Bât.3,3 rue Lavoisier, F-93500 Pantin (FR)**
Inventeur: **Grandadam, Jean André, 4, rue Lhomme, Saint-Maur-des-Fossés (FR)**
Inventeur: **Dreux, Huguette, 31, rue Pierret, Neuilly-sur-Seine (FR)**

(74) Mandataire: **Tonnellier, Marie-José et al, ROUSSEL-UCLAF 111, route de Noisy Boîte Postale no.9, F-93230 Romainville (FR)**

EP 0 053 978 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Nouveaux dérivés de l'estra 4,9-diène, leur procédé de préparation et leur application comme médicaments

La présente invention concerne de nouveaux dérivés de l'estra 4,9-diène, leur procédé de préparation, leur application comme médicaments et les compositions les renfermant.

L'invention a pour objet les composés de formule (I):

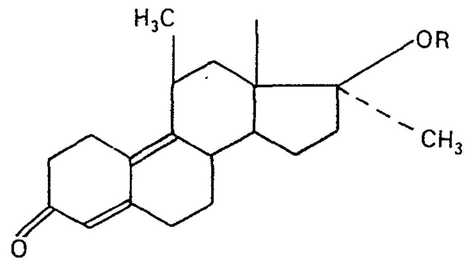

(I)

dans laquelle R représente un atome d'hydrogène, un radical alkyle renfermant de 1 à 8 atomes de carbone ou un radical acyle renfermant de 1 à 18 atomes de carbone.

Lorsque R représente un radical alkyle, il s'agit de préférence du radical méthyle, éthyle, propyle, isopropyle, butyle, isobutyle ou terbutyle.

Lorsque R représente un radical acyle, on entend de préférence par acyle le reste d'un acide aliphatique ou cycloaliphatique saturé ou insaturé et notamment le reste d'un acide alcanoïque tel que par exemple l'acide acétique, propionique, butyrique ou isobutyrique ou undécylique; le reste d'un acide hydroxy alcanoïque tel que par exemple l'acide hydroxy acétique; le reste d'un acide cycloalcoylcarboxylique ou (cycloalcoyl) alcanoïque tel que par exemple l'acide cyclopropyl, cyclopentyl ou cyclohexylcarboxylique, cyclopentyl ou cyclohexyl acétique ou propionique, le reste d'un acide benzoïque ou d'un acide phénylalcanoïque tel que l'acide phényl acétique ou phényl propionique; le reste d'un amino acide tel que l'acide diéthylamino acétique ou aspartique ou le reste de l'acide formique.

Les produits de formule (I) font partie d'une famille de composés revendiqués par exemple dans le brevet français 2 377 418. Toutefois aucun des produits de formule (I) n'a été décrit à ce jour, ni dans la demande précitée, ni ailleurs.

On vient de découvrir que les produits de formule (I) présentaient des propriétés pharmacologiques remarquables notamment une très intéressante activité anabolisante et androgène. Cette activité anabolisante et androgène exceptionnelle est totalement inattendue par rapport à celle des produits de structure chimique voisine décrite par exemple dans le brevet français 2 377 418. La partie expérimentale exposée ci-après met clairement en évidence le caractère exceptionnel des propriétés pharmacologiques.

L'invention a plus particulièrement pour objet le composé répondant à la formule (I) dans laquelle R représente un atome d'hydrogène.

Les produits de formule (I) présentent comme il a été indiqué ci-dessus de remarquables propriétés pharmacologiques, notamment une activité anabolisante et androgène exceptionnelle.

Les produits de l'invention peuvent être utilisés dans le traitement de l'andropause, du syndrome adiposogénital, des métrorragies fonctionnelles, du fibrome et des endométrioses ainsi que dans celui des asthénies, ostéoporoses, sénescences et perturbations métaboliques après traitement prolongé par corticothérapie.

L'invention a donc pour objet, à titre de médicament, les produits de formule (I) et tout particulièrement le produit dont la préparation est donnée en exemple.

La posologie utile varie en fonction de l'affection à traiter et de la voie d'administration, elle peut varier par exemple de 0,1 mg à 2 mg par jour chez l'adulte par voie orale.

Les médicaments de l'invention peuvent également être utilisés en médecine vétérinaire, pour augmenter la résistance générale organique aux agressions de toutes sortes, pour lutter contre les retards de croissance, l'amaigrissement, les troubles organiques généraux liés à un état de sénescence et également pour lutter, à titre secondaire, contre les maladies infectieuses, parasitaires et nutritionnelles ou encore pour promouvoir la prise de poids chez les animaux d'élevage.

Les médicaments vétérinaires de l'invention peuvent être administrés chez tous les animaux, notamment chez les animaux d'élevage comme les bovins, les porcins, les ovins et la volaille.

L'invention a donc notamment pour objet les composés de formule (I), à titre de médicaments vétérinaires. L'invention a plus particulièrement, pour objet, à titre de médicament vétérinaire, le produit dont la préparation est donnée en exemple.

Les composés de formule (I) sont utilisés par voie buccale, rectale, percutanée ou intraveineuse. Ils peuvent être prescrits sous forme de comprimés, de comprimés enrobés, de cachets, de capsules, de granulés, d'émulsions, de sirops, de suppositoires, de solutés et de suspensions injectables.

Les médicaments vétérinaires de l'invention peuvent être déposés sous forme d'implant dans le derme, de préférence à la base de l'oreille. Ces implants peuvent être administrés également dans le cou des animaux ou dans les muscles fessiers. Ils sont déposés par exemple de 20 jours à 4 mois avant l'abattage, de préférence de 1 à 3 mois avant celui-ci.

La posologie utile pour obtenir de bons résultats chez le veau peut varier de 50 à 400 mg sous forme d'un ou plusieurs traitements percutanés par exemple; on obtient en particulier d'excellents résultats chez le veau en effectuant deux traitements percutanés espacés l'un de l'autre de 5 à 10 jours, en administrant une quantité de principe actif variant de 150 à 250 mg. On peut par

exemple administrer par voie percutanée 2 fois 200 mg de produit de l'exemple 1 à une semaine d'intervalle.

L'invention a également pour objet les compositions pharmaceutiques renfermant comme principe actif au moins un produit de formule (I).

Le ou les principes actifs peuvent y être incorporés à des excipients habituellement employés dans les compositions pharmaceutiques à usage humain ou dans les compositions pharmaceutiques à usage vétérinaire, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de l'invention peuvent être également utilisés en tant qu'additifs à l'alimentation animale pour promouvoir la prise de poids chez les animaux d'élevage; les produits de l'invention sont alors associés à un mélange nutritif adapté à l'alimentation animale. Le mélange nutritif utilisé peut renfermer des céréales, des sucres et graisses, des tourteaux de soja, d'arachide et de tournesol, des farines d'origine animale, par exemple des farines de poissons, des acides aminés de synthèse, des sels minéraux, des vitamines et des anti-oxydants.

L'invention a également pour objet un procédé de préparation des produits de formule (I), caractérisé en ce que l'on soumet un composé de formule (II)

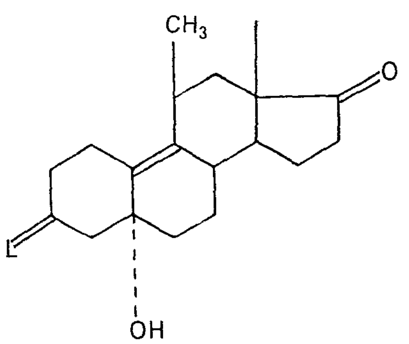

(II)

dans laquelle L représente un groupement cétal, à l'action d'un agent de méthylation puis à celle d'un agent de déshydratation capable en même temps de libérer la fonction cétone, pour obtenir la 11β-méthyl 17β-hydroxy 17α-méthyl estra 4,9-dien 3-one, c'est-à-dire le composé de formule (I) dans laquelle R représente de l'hydrogène, que l'on soumet si désiré à l'action d'un agent d'éthérification ou d'estérification.

Par cétal, on entend un groupement alkylcétalcyclique ayant de 2 à 4 atomes de carbone et notamment l'éthylène cétal ou le propylène cétal, ou bien un dialcoylcétal comme par exemple le diméthyl ou le diéthylcétal.

Comme agent de méthylation, on utilise de préférence un halogénure de méthylmagnésium, par exemple un bromure ou un chlorure de méthylmagnésium.

L'agent de déshydratation capable de libérer la fonction cétone est une résine sulfonique (forme acide), par exemple une résine sulfonique du commerce à support de polystyrène ou à support de copolymère styrène/divinylbenzène, mais on peut également utiliser un acide minéral tel que l'acide chlorhydrique ou l'acide sulfurique dans un alcanol inférieur ou l'acide perchlorique dans l'acide acétique, ou un acide sulfonique comme l'acide paratoluène sulfonique.

L'éthérification et l'estérification sont réalisées selon les méthodes classiques:

a) l'éthérification peut être réalisée par exemple en faisant réagir un halogènure d'alkyle sur le composé de formule:

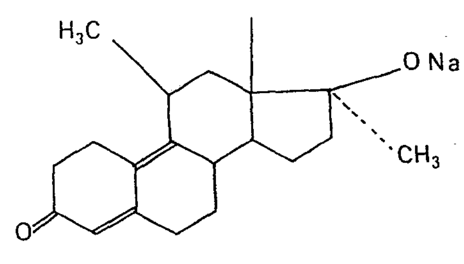

préparé par exemple en faisant réagir le sodium sur le composé de formule (I) dans lequel R = H.

Dans une variante évidente du procédé de l'invention, l'éthérification peut se faire également selon le schéma suivant:

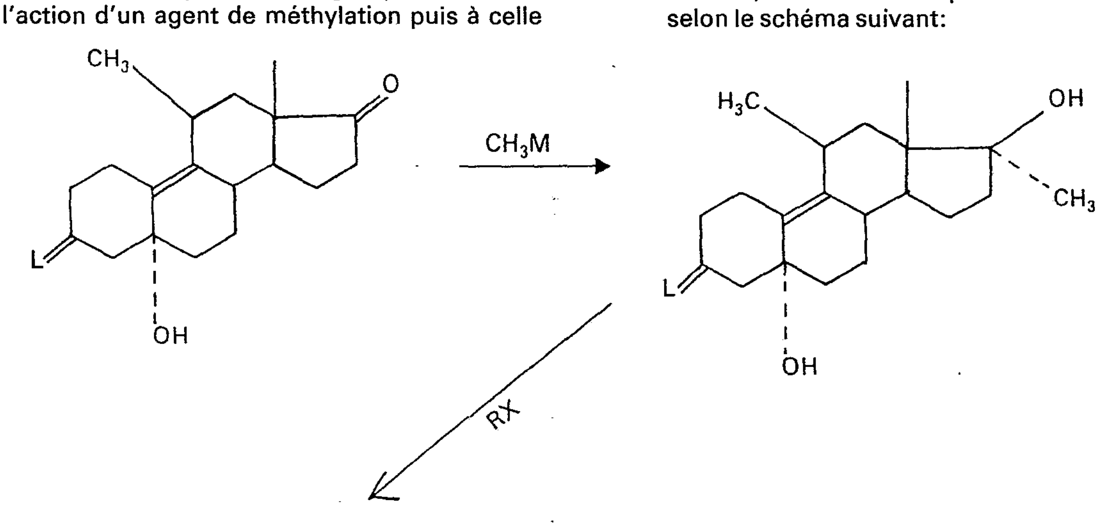

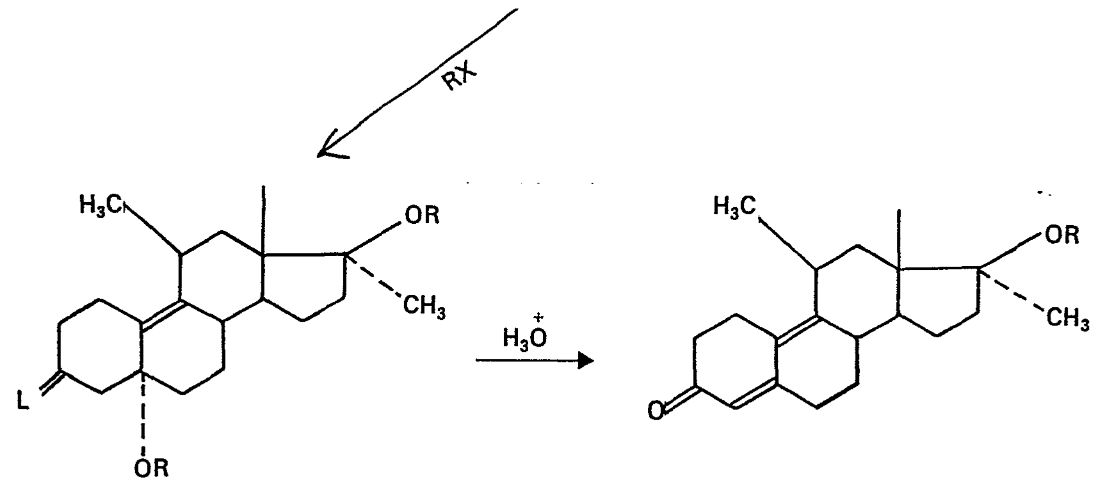


- M représentant un cation métallique ou Mg $X_1$,
- $X_1$ représentant un atome d'halogène,
- X représentant un atome d'halogène.

b) l'estérification peut être réalisée en faisant réagir le composé de formule (I) dans laquelle R représente de l'hydrogène avec le dérivé fonctionnel d'acide, par exemple un chlorure ou un anhydride d'acide.

Les composés de formule (II) utilisés comme produits de départ peuvent être préparés selon le procédé indiqué dans le brevet français 2 377 417.

Les exemples suivants illustrent l'invention sans toutefois la limiter.

Exemple 1:

11β-méthyl 17β-hydroxy 17α-méthyl estra 4,9-dièn 3-one.

Dans une solution éthérée 2,25 M de bromure de méthyle magnésium, sous agitation et sous atmosphère inerte ou introduit régulièrement en 40 minutes, en laissant la température s'élever, une solution de 100 g de 3,3-diméthoxy 5α-hydroxy 11β-méthyl estr-9(10)-ène 17-one préparé selon le procédé indiqué dans le brevet belge 862 868 dans 700 cm³ de dichloroéthane. On maintient à 40°C ±1 sous atmosphère inerte et sous agitation pendant 1 heure 45 minutes. Après refroidissement à 20°C, on verse le mélange réactionnel dans 600 cm³ d'eau, de glace et 400 g de chlorure d'ammonium. On agite 10 minutes, décante, extrait au chloroforme, lave à l'eau, sèche et concentre à sec sous pression réduite. On effectue un entraînement par 100 cm³ d'éthanol à 95°. On dissout le produit cristallisé obtenu, dans 836 cm³ d'éthanol, ajoute 83,6 g de résine «Rédex CF» et agite au reflux pendant 3 heures. On filtre à 65°C et concentre à sec le filtrat. On effectue un entraînement à l'acétone. On reprend le résidu huileux dans l'acétone, glace à −10°C, essore, rince à l'acétone à −20°C et obtient 25 g de produit brut. Après recristallisation dans l'acétate d'éthyle on obtient un produit fondant à 157,5°C.

$[\alpha]_D = -141{,}5°$ (c = 1% chloroforme).

Exemple 2:

Etude de l'activité androgène.

L'activité androgène du produit de l'exemple 1 a été étudiée en comparaison avec le produit analogue 11β n-propyl, produit A, selon la méthode des récepteurs hormonaux décrite par JP Raynaud et Coll dans «J. Ster. Biochem» 1975 6 - 615 622.

La technique est la suivante:

La prostate, prélevée sur des rats mâles castrés 24 heures auparavant, est homogénéisée dans un tampon trométhamine 10 millimoles, saccharose 0,25 M, HCl pH 7,4.

L'homogénat est centrifugé à 105 000 g pendant une heure. Le liquide surnageant ou «cytosol» est alors ajusté de façon à avoir une dilution 1/5 (poids/volume).

On incube à 0°C pendant deux heures, le cytosol avec une concentration fixe de 17β-hydroxy 17β-méthyl estra 4,9,11-triène 3-one tritié, désigné ci-après par produit R tritié, en présence ou non de concentration croissante de ce même produit froid, désigné ci-après produit R froid, de testostérone ou de produit à tester.

On détermine au bout de deux heures la radioactivité du produit tritié lié au récepteur par la technique d'adsorption au charbon-dextran (1,25%-0,625%).

On trace ensuite:

- les courbes représentant les pourcentages de produit R tritié lié en fonction du log de la concentration, du produit R froid, de la testostérone ou du produit à tester ajoutés,
- la droite $I_{50}$ parallèle à l'axe des abscisses et d'ordonnée

$$\frac{B}{T} = \frac{B/T\ Max. + B/T\ Min.}{2}$$

B/T Max. est le pourcentage de produit R tritié lié quand aucun produit n'est ajoutée,

B/T Min. est le pourcentage de produit R tritié lié quand la quantité maximale de produit R froid est ajoutée.

Les intersections de cette droite $I_{50}$ et des courbes permettent de déterminer les valeurs CT et CX.

CT: concentration de la testostérone froide qui inhibe de 50% la fixation du produit R tritié,

CX: concentration du produit testé qui inhibe de 50% la fixation du produit R tritié.

L'affinité relative du produit testé ou ARL est donnée par la formule:

$$ARL = 100 \times \frac{CT}{CX}$$

Les résultats obtenue sont les suivants:

| Produit | ARL |
|---|---|
| Testostérone | 100 |
| Produit de l'exemple 1 | 90 |
| Produit A | 22 |

Conclusion:

Le produit de l'exemple 1 présente une affinité pour le récepteur androgène beaucoup plus importante que celle du produit de l'art antérieur.

Exemple 3:

Effet sur le bétail du produit de l'exemple 1.

A - L'essai a été effectué sur des veaux de race FFPN. Les animaux sont engraissés en recevant tous la même quantité de nourriture, et sont divisés en 2 lots:
- un lot témoin,
- un lot auquel on administre au jour J = 0 et au jour J + 7 200 mg du produit de l'exemple 1 par voie percutanée. On pèse les animaux 7 jours avant le premier traitement, le jour du premier traitement J = 0, le jour du deuxième traitement (J + 7), une semaine après le deuxième traitement (J + 14).

Les résultats obtenus sont les suivants:

| | témoins | traités |
|---|---|---|
| Poids moyen des animaux au jour J - 7 | 45,23 kg | 45,50 kg |
| Poids moyen des animaux au jour J = 0 | 52,10 kg | 52,30 kg |
| Poids moyen des animaux au jour J + 7 | 59,42 kg | 60,47 kg |
| Poids moyen des animaux au jour J + 14 | 67,78 kg | 70,67 kg |
| Gain de poids moyen | 15,68 kg | 18,37 kg |

B - Mise en évidence d'un effet retard

Pendant les sept jours qui suivent l'arrêt du traitement entre les jours J 14 et J 21, les veaux témoins ont eu un gain de poids moyen de 6,28 kg alors que les traités ont eu un gain de poids moyen de 8,63 kg.

Conclusion:

L'administration du produit de l'exemple 1 a permis d'améliorer très nettement le gain du poids moyen.

Exemple 4:

1) Exemples de compositions pharmaceutiques.

On a préparé un implant renfermant:
Produit de l'exemple 1 140 mg
excipient q.s. pour un implant

On a préparé une pommade renfermant:
Produit de l'exemple 1 40 mg
excipient q.s. pour 1 g

**Revendications**

1) Les composés de formule (I)

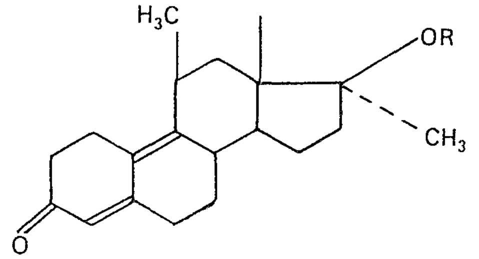

(I)

dans laquelle R représente un atome d'hydrogène ou un radical alkyle renfermant de 1 à 8 atomes de carbone ou un radical acyle renfermant de 1 à 18 atomes de carbone.

2. Le composé de formule (I) tel que défini à la revendication 1, dans laquelle R représente un atome d'hydrogène.

3. A titre de médicament, les composés définis à la revendication 1.

4. A titre de médicament, le composé défini à la revendication 2.

5. A titre de médicaments vétérinaires, les composés définis à la revendication 1.

6. A titre de médicament vétérinaire, le composé défini à la revendication 2.

7. Les compositions pharmaceutiques renfermant comme principe actif au moins un médicament défini à l'une quelconque des revendications 3 à 6.

8. A titre d'additif à l'alimentation animale, les composés de formule (I) tels que définis à la revendication 1 ou 2.

9. Procédé de préparation des composés de formule (I) tels que définis à la revendication 1 ou 2, caractérisé en ce que l'on soumet un composé de formule (II)

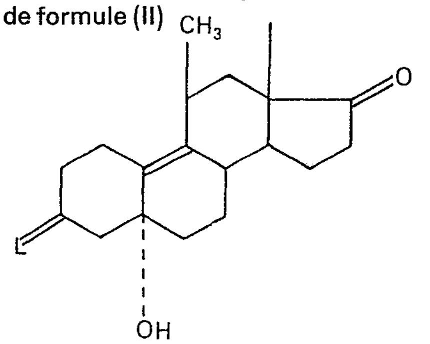

(II)

dans laquelle L représente un groupement cétal, à l'action d'un agent de méthylation puis à celle d'un agent de déshydratation capable en même temps de libérer la fonction cétone, pour obtenir la 11β-méthyl 17β-hydroxy 17α-méthyl estra 4,9-dien 3-one, c'est-à-dire le composé de formule (I) dans laquelle R représente de l'hydrogène, que l'on soumet si désiré à l'action d'un agent d'éthérification ou d'estérification.

**Patentansprüche**

1. Verbindungen der Formel I

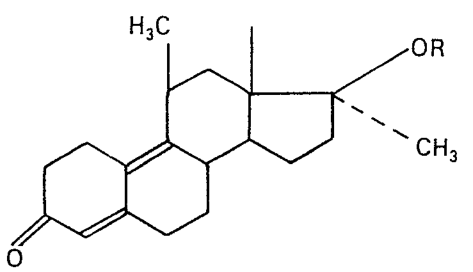

(I)

worin R ein Wasserstoffatom oder einen Alkylrest mit 1 bis 8 Kohlenstoffatomen oder einen Acylrest mit 1 bis 18 Kohlenstoffatomen bedeutet.

2. Verbindung der Formel I gemäss Anspruch 1, worin R ein Wasserstoffatom bedeutet.

3. Als Arzneimittel die Verbindungen gemäss Anspruch 1.

4. Als Arzneimittel die Verbindung gemäss Anspruch 2.

5. Als veterinäre Arzneimittel die Verbindungen gemäss Anspruch 1.

6. Als veterinäres Arzneimittel die Verbindung gemäss Anspruch 2.

7. Pharmazeutische Zusammensetzungen, enthaltend als Wirkstoff zumindest ein Arzneimittel gemäss einem der Ansprüche 3 bis 6.

8. Als Additiv für die tierische Ernährung die Verbindungen der Formel I gemäss Anspruch 1 oder 2.

9. Verfahren zur Herstellung von Verbindungen der Formel I gemäss Anspruch 1 oder 2, dadurch gekennzeichnet, dass man eine Verbindung der Formel II

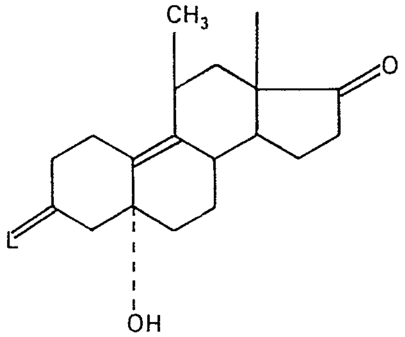

(II)

worin L eine Ketalgruppe bedeutet, der Einwirkung eines Methylierungsmittels und danach derjenigen eines Dehydratationsmittels, das befähigt ist, gleichzeitig die Ketonfunktion freizusetzen, unterzieht, um das 11β-Methyl-17β-hydroxy-17α-methyl-östra-4,9-dien-3-on, nämlich die Verbindung der Formel I zu erhalten, worin R ein Wasserstoffatom bedeutet, welche man gewünschtenfalls der Einwirkung eines Verätherungs- oder Veresterungsmittels unterzieht.

**Claims**

1. The compounds with the formula (I):

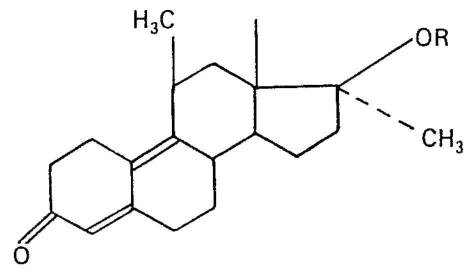

(I)

in which R represents a hydrogen atom or an alkyl radical containing from 1 to 8 carbon atoms or an acyl radical containing from 1 to 18 carbon atoms.

2. The compound with the formula (I) as defined in Claim 1, in which R represents a hydrogen atom.

3. As medicament, the compounds defined in Claim 1.

4. As medicament, the compound defined in Claim 2.

5. As veterinary medicaments, the compounds defined in Claim 1.

6. As veterinary medicament, the compound defined in Claim 2.

7. The pharmaceutical compositions containing as active principle one medicament at least as defined in any one of the Claims 3 to 6.

8. As additive to animal feed-stuff, the compounds with the formula (I) as defined in Claim 1 or 2.

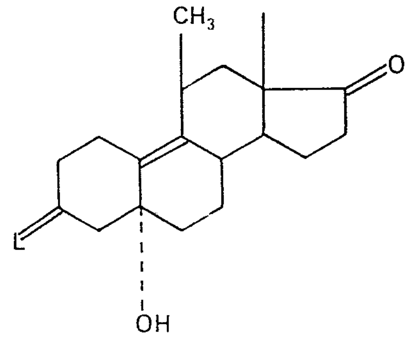

(II)

9. Preparation process for the compounds with the formula (I) as defined in Claim 1 or 2, characterized in that a compound with the formula (II):

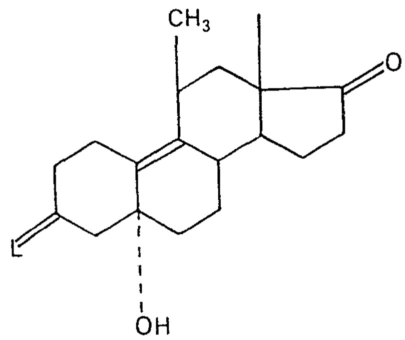

(II)

in which L represents a ketal group, is submitted to the action of a methylating agent then to that of a dehydratation agent able at the same time to liberate the ketone function, so as to obtain 11β-methyl 17β-hydroxy 17α-methyl estra 4,9-dien 3-one, i.e., the compound with the formula (I) in which R represents hydrogen, which, if desired, is submitted to the action of an etherification or esterification agent.